# EUROPÄISCHE PATENTANMELDUNG

(11) **EP 2 157 763 A1**
(43) Veröffentlichungstag der Anmeldung: **24.02.2010**
(21) Anmeldenummer: 09010621.2
(22) Anmeldetag: 18.08.2009
(51) Int. Cl.: H04L 29/08, H04L 29/06, H04B 7/185

(54) **Vorrichtung und Anordnung zum Erfassen oder Auswerten medizinischer Daten an Bord eines Verkehrsmittels**

(30) Priorität: 19.08.2008 DE 102008038312
(71) Anmelder: Lufthansa Technik AG, 22335 Hamburg (DE)
(72) Erfinder: Muirhead, Andrew, 22848 Norderstedt (DE)
(74) Vertreter: Glawe, Delfs, Moll

(57) **Zusammenfassung**

Gegenstand der Erfindung ist eine Vorrichtung zum Erfassen und oder Auswerten medizinischer Daten an Bord eines Verkehrsmittels. Sie weist folgende Merkmale auf:
a) wenigstens eine Schnittstelle für die Erfassung und/oder Eingabe medizinischer Daten;
b) eine Einrichtung zur Übermittlung medizinischer Daten und/oder Audio- und/oder Videodaten von der Vorrichtung an eine und/oder von einer außerhalb des Verkehrsmittels befindliche/n Bodenstation;
c) die Vorrichtung weist eine erste Betriebsart zur Übermittlung medizinischer Daten und/oder Audio- und/oder Videodaten von der Vorrichtung an eine und/oder von einer außerhalb des Verkehrsmittels befindliche/n Bodenstation auf, in der sie zur Sicherung einer für eine Videoübertragung ausreichenden Bandbreite auf den verwendeten Datenübertragungsmitteln ausgebildet ist;
d) die Vorrichtung weist eine zweite Betriebsart zur Übermittlung medizinischer Daten und/oder Audio- und/oder Videodaten von der Vorrichtung an eine und/oder von einer außerhalb des Verkehrsmittels befindliche/n Bodenstation auf, in der sie zur Datenübertragung eine schmalere Bandbreite als in der ersten Betriebsart und/oder eine diskontinuierliche Datenübertragungsart (packet mode) verwendet.

## Beschreibung

Die Erfindung betrifft eine Vorrichtung sowie eine Anordnung zum Erfassen oder Auswerten medizinischer Daten an Bord eines Verkehrsmittels.

In abgelegenen Orten, insbesondere Verkehrsmitteln wie Flugzeugen, Schiffen oder dergleichen kann es zu medizinischen Notfällen kommen, ohne dass unmittelbar kompetente ärztliche Hilfe verfügbar ist. Tritt beispielsweise an Bord eines Flugzeugs im Reiseflug ein medizinischer Notfall auf, kann das Kabinenpersonal erste Hilfe leisten, gegebenenfalls kann auch ein unter Passagieren an Bord anwesender Arzt hinzugezogen werden.

Für das Kabinenpersonal eines Flugzeugs kann es schwierig sein, eine Entscheidung darüber zu treffen, ob die Ernsthaftigkeit des medizinischen Zwischenfalls oder Notfalls eine außerplanmäßige Landung am nächstgelegenen Flughafen erfordert, in dem medizinische Hilfe verfügbar ist. Da eine solche außerplanmäßige Landung mit hohen Kosten sowie Unannehmlichkeiten für die übrigen Passagiere verbunden ist, ist es beispielsweise aus WO 03/030405 A1 bereits bekannt, medizinische Daten der betreffenden Person zu erfassen und über ein sogenanntes Telemedizinmodul diese Daten einer Bodenstation zu übermitteln, wo sie von einem Arzt ausgewertet werden können. Ferner kann über dieses Telemedizinmodul eine Kommunikation mit einem Arzt am Boden erfolgen.

Der Erfindung liegt die Aufgabe zugrunde, eine Vorrichtung sowie eine Anordnung der eingangs genannten Art zu schaffen, die eine effiziente und kostengünstige Kommunikation zwischen der an Bord befindlichen Vorrichtung zum Erfassen oder Auswerten medizinischer Daten sowie einer Bodenstation (einer sogenannten Telemedizinstation am Boden) ermöglicht.

Die erfindungsgemäße Vorrichtung weist folgende Merkmale auf:
a) wenigstens eine Schnittstelle für die Erfassung und/oder Eingabe medizinischer Daten;
b) eine Einrichtung zur Übermittlung medizinischer Daten und/oder Audio- und/oder Videodaten von der Vorrichtung an eine und/oder von einer außerhalb des Verkehrsmittels befindliche/n Bodenstation;
c) die Vorrichtung weist eine erste Betriebsart zur Übermittlung medizinischer Daten und/oder Audio- und/oder Videodaten von der Vorrichtung an eine und/oder von einer außerhalb des Verkehrsmittels befindliche/n Bodenstation auf, in der sie zur Sicherung einer für eine Videoübertragung ausreichenden Bandbreite auf den verwendeten Datenübertragungsmitteln ausgebildet ist;
d) die Vorrichtung weist eine zweite Betriebsart zur Übermittlung medizinischer Daten und/oder Audio- und/oder Videodaten von der Vorrichtung an eine und/oder von einer außerhalb des Verkehrsmittels befindliche/n Bodenstation auf, in der sie zur Datenübertragung eine schmalere Bandbreite als in der ersten Betriebsart und/oder eine diskontinuierliche Datenübertragungsart (packet mode) verwendet.

Zunächst seien einige im Rahmen der Erfindung verwendete Begriffe erläutert.

Eine Vorrichtung zum Erfassen und oder Auswerten medizinischer Daten ist eine vorzugsweise tragbare Vorrichtung, in die medizinische Daten über wenigstens eine Schnittstelle eingegeben werden können und die diese Daten entweder unmittelbar oder gegebenenfalls in einer aufbereiteten oder ausgewerteten Form an eine Bodenstation übermitteln kann. Bei den medizinischen Daten kann es sich insbesondere um Messdaten von Sensoren für Vitalfunktionen (beispielsweise Blutdruck, Puls, pulsoximetrische Daten, EKG-Daten oder dergleichen) handeln, ferner können medizinische Daten von einer Hilfsperson über eine geeignete Schnittstelle wie beispielsweise eine Tastatur, einen Touchscreen oder dergleichen eingegeben werden.

Der Begriff "an Bord eines Verkehrsmittels" ist weit zu verstehen und umfasst somit Vorrichtungen, die in fahrenden und oder fliegenden Verkehrsmitteln, vorzugsweise auch in Flugzeugen, verwendbar sind. Es versteht sich, dass eine erfindungsgemäße Vorrichtung auch an sonstigen Orten verwendet werden kann, an denen ärztliche Hilfe nicht ohne weiteres verfügbar ist. Beispielhaft genannt seien einsam gelegene Ortschaften, Inseln, Explorationsplattformen im Wasser oder dergleichen.

Die erfindungsgemäße Vorrichtung (nachfolgend auch Telemedizinmodul genannt) weist wenigstens zwei Betriebsarten auf, in denen sie auf unterschiedliche Art und Weise mit der Bodenstation kommuniziert. In einer ersten Betriebsart stellen die zur Kommunikation mit der Bodenstation verwendeten Datenübertragungsmittel eine Bandbreite zur Verfügung, die für eine Videoübertragung von der bzw. an die Bodenstation ausreicht. In dieser Betriebsart kann beispielsweise Kabinenpersonal mittels des Telemedizinmoduls unmittelbar mit einem Arzt in der Bodenstation sprechen (Audioübertragung), zusätzlich können dabei mittels Videoübertragung beispielsweise Echtzeitbilder des Patienten an den Boden übertragen werden oder der Arzt in der Bodenstation kann dem Kabinenpersonal Handlungsanweisungen übermitteln und per Video demonstrieren. In dieser ersten Betriebsart belegt die Vorrichtung eine verhältnismäßig hohe Bandbreite, die beispielsweise bei Inanspruchnahme von Satellitenverbindungen in einem fliegenden Flugzeug recht kostspielig sein kann und zudem die Nutzung der entsprechenden Satellitenverbindung durch andere Nutzer an Bord des Flugzeugs (beispielsweise Betriebssysteme des Flugzeugs, die Wartungsdaten übermitteln oder Passagiere, die E-Mail übermitteln oder im Internet arbeiten) beeinträchtigen kann, da in aller Regel die allen Nutzern an Bord eines Verkehrsmittels bei einer Satellitenverbindung zur Verfügung stehende Bandbreite beschränkt ist.

Erfindungsgemäß weist die Vorrichtung daher eine zweite Betriebsart auf, in der sie eine schmalere Bandbreite als in der ersten Betriebsart in Anspruch nimmt und/oder eine diskontinuierliche Datenübertragungsart (beispielsweise packet mode) in Anspruch nimmt. Die Erfindung hat erkannt, dass bei vielen medizinischen Notfällen eine breitbandige Verbindung zum Boden, die eine Übermittlung von Videodaten in Echtzeit erlaubt, entweder nicht oder jedenfalls nicht permanent erforderlich ist. Wenn beispielsweise lediglich eine Audiokommunikation mit der Bodenstation erforderlich ist, muss lediglich eine deutlich geringere Bandbreite in Anspruch genommen werden. Ferner kann es eine Situation geben, in der das Kabinenpersonal erste Hilfe geleistet hat und mittels des Telemedizinmoduls lediglich eine weitere Aufzeichnung von Vitaldaten des Patienten erfolgt, die an den Boden übermittelt werden sollen, um dort später zur Auswertung (beispielsweise zwecks Vorbereitung bzw. Durchführung einer weitergehenden medizinischen Behandlung) zur Verfügung zu stehen. In einer solchen Situation kann ebenfalls eine schmalbandige Datenverbindung oder gegebenenfalls eine diskontinuierliche Datenübertragung ausreichen. Die Erfindung erlaubt es somit, eine mit hohen Kosten und der Beeinträchtigung anderer Nutzer verbundene breitbandige Datenübertragung nur dann in Anspruch zu nehmen, wenn die Situation es gebietet. Eine geeignete Bandbreite für die zweite Betriebsart kann beispielsweise 32 kBit/s sein. Bei Einsatz eines entsprechenden Kompressions-Algorithmus für Audiodaten können unter Umständen auch noch niedrigere Bandbreiten gewählt werden.

Der Begriff Bodenstation bezeichnet im Rahmen der Erfindung eine Einrichtung, mittels der eine Datenverbindung mit dem Verkehrsmittel hergestellt werden kann und über die ein sogenannter Telemedizindienstleister mit dem Verkehrsmittel verbunden werden kann. Der Begriff Telemedizindienstleister bezeichnet eine Einrichtung, die zur Auswertung der übermittelten medizinischen Daten geeignet ist und bei Bedarf diagnostische oder therapeutische Anweisungen oder Ratschläge an Bord des Verkehrsmittels übermitteln kann. In aller Regel ist ein solcher Telemedizindienstleister mit einem oder mehreren Ärzten besetzt.

Die Schnittstellen für die Erfassungen oder Eingabe medizinischer Daten können Anschlüsse für Sensoren für Vitalfunktionen des Körpers umfassen. Solche Sensoren können beispielsweise EKG-Geräte, Pulsoximeter, Blutdruckmessgeräte, Körpertemperaturthermometer oder dergleichen umfassen. Es ist im Rahmen der Erfindung auch möglich, dass Schnittstellen vorhanden sind, über die das Telemedizinmodul bei Bedarf therapeutische Einrichtungen unmittelbar ansteuern kann. Beispielhaft genannt sei hier ein Defibrillator, der von dem Telemedizinmodul entweder automatisch anhand der ermittelten diagnostischen Daten oder manuell entweder durch Bedienungspersonal oder durch die Telemedizinstation am Boden angesteuert bzw. ausgelöst werden kann.

Es können ferner Schnittstellen für die Erfassung und oder Eingabe medizinischer Daten vorgesehen sein, die Audio- und/oder Videodaten erfassen oder zur Eingabe von Daten durch eine Hilfsperson ausgebildet sind, wobei eine solche Eingabe beispielsweise durch eine Tastatur, einen Touchscreen oder dergleichen erfolgen kann. Es kann insbesondere eine Schnittstelle für eine Videokamera vorgesehen sein, mittels der sich beispielsweise der Zustand eines Patienten dokumentieren lässt oder mit deren Hilfe in der Telemedizinstation am Boden eine Überprüfung der richtigen Durchführung diagnostischer oder therapeutischer Maßnahmen erfolgen kann. Ferner können Anschlüsse für ein Mikrofon, einen Lautsprecher oder Kopfhörer oder gegebenenfalls ein kombiniertes Headset vorgesehen sein, die eine Audiokommunikation mit der Telemedizinstation erleichtern.

Im Rahmen der Erfindung ist es möglich, dass zusätzliche Sensoren für Vitalfunktionen des Körpers vorgesehen sind, die nicht online mit dem Telemedizinmodul verbunden sind. Dabei kann es sich beispielsweise um ein übliches Fieberthermometer handeln, um eine Vorrichtung zur Bestimmung des Blutzuckers oder dergleichen. Diese Sensoren können durch entsprechend ausgebildetes Kabinenpersonal angewendet und die Messwerte dann manuell in das Telemedizinmodul eingegeben werden.

Die Datenübermittlung zwischen dem Telemedizinmodul und der Bodenstation erfolgt in der Regel in der Gestalt, dass zunächst mittels eines geeigneten Datenübertragungsnetzwerks im Verkehrsmittel eine Übertragung an eine Sende/Empfangseinrichtung des Verkehrsmittels erfolgt. Anschließend erfolgt eine in aller Regel drahtlose Übertragung (beispielsweise mittels einer breitbandigen Mikrowellenverbindung über Satelliten) an eine Bodenstation. Insbesondere bei schienengebundenen Verkehrsmitteln kann gegebenenfalls auch eine drahtgebundene Übertragung an die Bodenstation erfolgen. Innerhalb des Verkehrsmittels kann die Datenübertragung vom Telemedizinmodul in das Netzwerk des Verkehrsmittels entweder drahtgebunden oder bevorzugt drahtlos erfolgen, beispielsweise mittels eines bekannten Wireless Local Area Network (WLAN).

In der ersten Betriebsart kann erfindungsgemäß eine Bandbreite von wenigstens 64 kBit/s, vorzugsweise 64 bis 128 kBit/s, weiter vorzugsweise wenigstens 96 kBit/s vorgesehen sein. Eine solche Bandbreite ist in aller Regel hinreichend, um eine Videoübertragung in Echtzeit und hinreichender Qualität zu ermöglichen. Wird eine höhere Auflösung und oder Qualität einer Videoübertragung in Echtzeit gewünscht, kann eine entsprechend höhere Mindestbandbreite in der ersten Betriebsart vorgesehen sein.

In der zweiten Betriebsart kann erfindungsgemäß eine Bandbreite von wenigstens 16 kBit/s, vorzugsweise wenigstens 32 kBit/s, weiter vorzugsweise 32 bis 64 kBit/s vorgesehen sein.

Die Erfindung ermöglicht somit eine effiziente und andere Nutzer so wenig als möglich beeinträchtigende Nutzung der zur Verfügung stehenden Übertragungswege zwischen Flugzeug und Boden. Viele Satellitenübertragungsstrecken (beispielsweise die im Stand der Technik bekannten Inmarsat-Verbindungen) kann man beispielsweise im sogenannten Packet-Mode oder Circuit-Mode buchen. Im Packet-Mode wird der Datenverkehr nach übertragenem Datenvolumen bezahlt, es gibt jedoch in der Regel keine Garantie für eine jederzeit zur Verfügung stehende Mindestbandbreite oder Datenübertragungsrate. Im sogenannten Circuit-Mode wird eine vereinbarte Bandbreite fest und exklusiv zur Verfügung gestellt. Die Bezahlung erfolgt in der Regel nach der Zeitdauer der Nutzung bzw. des zur Verfügung Stellens dieser Bandbreite. Der Circuit-Mode ist daher in der Regel wesentlich teurer und wird aus Kostengründen nur dann verwendet, wenn eine bestimmte Mindestübertragungsqualität (beispielsweise bei der Übertragung von Videodaten) erreicht werden muss.

Viele seit längerem am Markt befindliche Satellitenübertragungsstrecken erreichen eine maximale Datenübertragungsrate zwischen Flugzeug und Boden von insgesamt beispielsweise 256 bis 512 kBit/s. Wird bei einer solchen Satellitenübertragungsstrecke ein Videodatensignal (in der Regel erforderliche Mindestbandbreite 64 kBit/s) im Packet-Mode übertragen, kommt es häufig auf Grund von Kommunikationskonflikten mit anderen Nutzern der Satellitenübertragungsstrecke (beispielsweise im gleichen Flugzeug sitzende und über die Satellitenübertragungsstrecke kommunizierende Passagiere) zu Verzögerungen in der Datenübertragung, so dass eine Echtzeit-Videoübertragung in hinreichender Qualität nicht möglich ist. In diesem Fall muss dann für die Videoübertragung auf den wesentlich teureren Circuit-Mode ausgewichen werden. Bei der schmalbandigeren zweiten Betriebsart wird hingegen in aller Regel der günstigere Packet-Mode ausreichen. Wenn aus Sicherheitsgründen auch die zweite Betriebsart im Circuit-Mode erfolgen soll, muss nur eine entsprechend geringere garantierte Bandbreite gebucht werden. Dies verringert die Kosten und belässt größere Teile der insgesamt zur Verfügung stehenden Bandbreite der Satellitenübertragungsstrecke zur Verfügung anderer Nutzer.

Es existieren breitbandigere Satellitenübertragungsstrecken, die bspw. das Ku-Band (12 bis 18 GHz) nutzen und beispielsweise von der Firma ViaSat angeboten werden. Solche Satellitenübertragungsstrecken erreichen Datenübertragungsraten im MBit-Bereich, beispielsweise 1,8 bis 10 MBit/s. Die zur Verfügung stehende Bandbreite vom Flugzeug zum Boden ist dabei häufig geringer als beim umgekehrten Weg. Bei entsprechend breitbandigen Satellitenübertragungsstrecken wird es im Rahmen der Erfindung häufig möglich sein, auch die Videodatenübertragung bei der ersten Betriebsart im Packet-Mode durchzuführen. Unter Umständen kann es jedoch auch hier sinnvoll sein, ebenfalls die erste Betriebsart auf der Satellitenübertragungsstrecke im Circuit-Mode arbeiten zu lassen, um auch bei hoher Auslastung der Satellitenkommunikationsstrecke eine einwandfreie und nicht verzögerte Videodatenübertragung zu erreichen.

Erfindungsgemäß kann vorgesehen sein, dass sich die erste Betriebsart automatisch einschaltet, wenn eine Videoübertragung vorgenommen wird. Auf diese Art und Weise wird sichergestellt, dass die Vorrichtung die entsprechende hohe Bandbreite für Videoübertragung nur dann in Anspruch nimmt, wenn tatsächlich eine Übertragung erfolgt.

Bevorzugt weist die Vorrichtung eine Identifizierungseinrichtung auf, die sie bei der Verwendung der ersten Betriebsart bei dem Betreiber der verwendeten Datenübertragungsmittel als zur bevorrechtigten Anforderung einer entsprechenden Bandbreite identifiziert. Beispielsweise kann die sogenannte MAC-ID oder der Netzwerkname der erfindungsgemäßen Vorrichtung im Netzwerk des Flugzeugs registriert sein bei dem Betreiber des Satellitennetzwerks, über das die Breitbandverbindung zwischen Flugzeug und Boden hergestellt wird. Loggt sich das Telemedizinmodul in das WLAN des Flugzeugs ein, wird dies über die Sende-/Empfangseinrichtung des Flugzeugs dem Betreiber des Satellitennetzwerks zur Kenntnis gebracht. Dieser bzw. das an Bord des Flugzeugs befindliche Routing-System trifft dann Vorkehrungen dafür, dass für eine Verbindung zwischen dem betreffenden Flugzeug und dem Boden im Satellitennetzwerk kurzfristig und bevorzugt die erforderliche Mindestbandbreite bereitgestellt werden kann. Gegebenenfalls müssen zu diesem Zweck andere in diesem Satellitennetzwerk bestehende Verbindungen in ihrer Bandbreite eingeschränkt oder getrennt werden, insbesondere bestehende Verbindungen zwischen dem betreffenden Flugzeug und dem Boden wie beispielsweise Internetverbindung an Bord befindlicher Passagiere oder Verbindungen von Betriebssystemen des Flugzeugs mit dem Boden zum Austausch von Wartungsdaten. Sobald dann die erste Betriebsart (Videoübertragung) eingeschaltet wird, werden die entsprechenden Maßnahmen getroffen um die erforderliche Mindestbandbreite sofort und unterbrechungsfrei bereitzustellen. War das Netzwerk vor Eintreten des medizinischen Notfalls nur gering belastet, kann es auch sein, dass zur Sicherstellung der erforderlichen Mindestbandbreite keinerlei Maßnahmen erforderlich sind. Im Rahmen der Erfindung ist es auch möglich, dass die Vorrichtung ein manuelles Bedienungselement aufweist, mittels der sich eine gewünschte Mindestbandbreite manuell vorgeben lässt. Auf diese Weise kann beispielsweise in der zweiten Betriebsart die zur Verfügung stehende Bandbreite manuell erhöht werden, wenn dem Bediener die Vorrichtung zu langsam reagiert.

Erfindungsgemäß kann die Vorrichtung eine dritte Betriebsart aufweisen, in der keine Datenübertragung von einer bzw. an eine Bodenstation stattfindet. In dieser sogenannten Stand Alone Betriebsart kann das Telemedizinmodul beispielsweise zur Erfassung diagnostischer Daten oder gegebenenfalls Therapie einfacherer medizinischer Zwischenfälle verwendet werden, die keine Notfallverbindung mit einem Telemedizindienstleister erfordern.

Die erfindungsgemäße Vorrichtung kann zusätzlich eine Einrichtung aufweisen, die einen Identifizierungscode für einen diagnostizierten bzw. behandelten Patienten ausgibt. Es kann sich beispielsweise um einen Labeldrucker handeln, der einen Barcode oder ein anderes Label auf ein Band oder dergleichen druckt, das den Patienten um das Handgelenk gelegt werden kann. Die während des medizinischen Zwischenfalls an den Boden übermittelten Daten können dort in einer Datenbank gespeichert werden. Wird der Patient nach der Landung oder Ankunft des Verkehrsmittels beispielsweise in ein Krankenhaus eingeliefert, kann dort der Code auf dem Label mittels eines geeigneten Lesegerätes gelesen werden, wodurch die in der Bodenstation gespeicherten Daten für das Personal des jeweiligen Krankenhauses beispielsweise über das Internet zugänglich gemacht werden können. Auf diese Weise kann einfach, verlässlich und gleichzeitig sicher auf die entsprechenden Daten zugegriffen werden, wenn im Anschluss an den medizinischen Zwischenfall eine weitere Behandlung erfolgt.

Gegenstand der Erfindung ist ferner eine Anordnung zum Erfassen oder Auswerten medizinischer Daten an Bord eines Verkehrsmittels, die aufweist:
a) an Bord des Verkehrsmittels eine Vorrichtung nach einem der Ansprüche 1 bis 8;
b) an Bord des Verkehrsmittels ein Netzwerk zur Übertragung der Daten von der Vorrichtung an eine Sende/Empfangseinrichtung des Verkehrsmittels und umgekehrt;
c) eine Bodenstation mit einer Sende/Empfangseinrichtung, die zur Datenkommunikation mit der Sende/Empfangseinrichtung des Verkehrsmittels ausgebildet ist;
d) in der Bodenstation Einrichtungen zur Datenkommunikation mit einer Einrichtung zur Erbringung telemedizinischer Dienstleistungen.

Wie bereits im Kontext der Erläuterung der erfindungsgemäßen Vorrichtung geschildert, kann als Netzwerk an Bord des Verkehrsmittels ein drahtloses Netzwerk wie beispielsweise ein WLAN verwendet werden; die Datenkommunikation zwischen Verkehrsmittel und Bodenstation kann bevorzugt mittels einer breitbandigen Funkverbindung wie beispielsweise einer Mikrowellenverbindung über Satelliten erfolgen.

In der Regel wird die Bodenstation die entsprechenden Daten an einen Telemedizindienstleister weiterleiten. Dies kann entweder über proprietäre Datenleitungen oder über ein öffentliches Netzwerk wie das Internet erfolgen. Bei der Benutzung öffentlicher Netzwerke kann die Datenkommunikation bevorzugt verschlüsselt erfolgen, da hier zu schützende persönliche Daten übertragen werden.

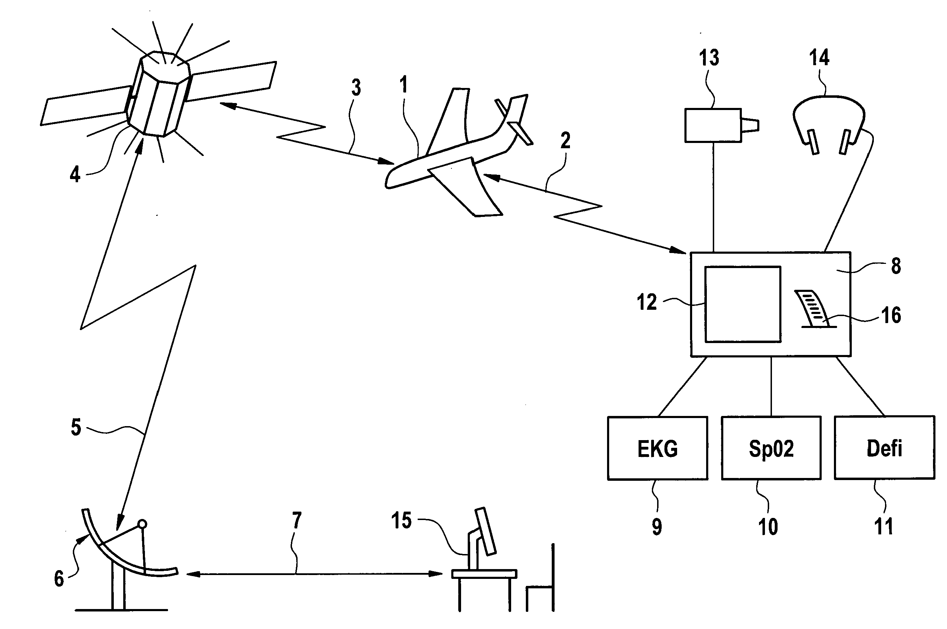

Eine Ausführungsform der Erfindung wird im Folgenden anhand der Zeichnung beschrieben, die schematisch eine erfindungsgemäße Anordnung zeigt.

An Bord eines Flugzeugs 1 ist ein Wireless Local Areal Network (WLAN) 2 installiert. Das Flugzeug hat im Reiseflug über einen Satelliten 4 eine Verbindung 3, 5 mit einer Bodenstation 6. Diese Bodenstation 6 ist mit dem Internet 7 verbunden.

Im normalen Reiseflug kann über die Satellitenverbindung 3, 4, 5 eine ständige Datenverbindung mit der Bodenstation 6 und damit mit dem Internet 7 aufrechterhalten werden. Diese Verbindung kann von den Passagieren des Flugzeugs 1 zur Kommunikation genutzt werden, ferner kann sie von Betriebssystemen des Flugzeugs zur Übermittlung von Wartungsdaten oder dergleichen verwendet werden. Über das WLAN 2 im Inneren des Flugzeugs erfolgt eine Verbindung der Endgeräte der Benutzer mit einer geeigneten (nicht dargestellten) Sende/- Empfangseinrichtung des Flugzeugs 1, die eine Verbindung 3 mit dem Kommunikationssatelliten 4 ermöglicht.

Tritt ein medizinischer Zwischenfall an Bord auf, kann das Kabinenpersonal ein üblicherweise in einem Notfallkoffer oder dergleichen bereitgehaltenes Telemedizinmodul 8 aktivieren. Nach der Aktivierung loggt sich das Telemedizinmodul 8 in das WLAN 2 des Flugzeugs 1 ein und wird dort mittels seiner MAC-ID identifiziert. Diese Identifizierung bewirkt, dass die Sende-/Empfangseinrichtung des Flugzeugs 1 über den Satelliten 4 an die Bodenstation 6 den Eintritt eines medizinischen Zwischenfalls meldet. Der Betreiber der Satellitenverbindung 3, 4, 5 sorgt dann dafür, dass von diesem Zeitpunkt an eine ausreichende Bandbreite auch zur Übertragung von Videodaten in Echtzeit über die Verbindung 3, 4, 5 entweder sofort bereit gestellt wird oder aber Vorkehrungen dafür getroffen werden, dass diese Bandbreite unmittelbar bereit gestellt werden kann, wenn an dem Telemedizinmodul 8 die erste Betriebsart (Videoübertragung) tatsächlich aktiviert wird.

Das Kabinenpersonal kann Sensoren für Vitalfunktionen des Körpers am Patienten einrichten und mit dem Telemedizinmodul 8 verbinden. In der Zeichnung sind beispielhaft dargestellt ein EKG-Modul 9, ein Pulsoximeter 10 sowie ein Defibrillator 11. Bei dem Defibrillator 11 handelt es sich um ein Therapiemodul, er kann zusätzlich Sensoren wie beispielsweise ein Einkanal EKG aufweisen. Die Verbindung zwischen den Sensoren bzw. Therapiemodulen 9, 10, 11 (und anderen nicht dargestellten Sensor- oder Therapiemodulen) mit dem Telemedizinmodul 8 kann entweder drahtgebunden oder drahtlos durch eine geeignete Funk- oder Infrarotverbindung erfolgen, beispielhaft genannt seien hier Bluetooth oder WLAN-Verbindungen.

Das Telemedizinmodul weist einen bei 12 schematisch dargestellten Touchscreen auf, ferner kann daran eine Videokamera 13 sowie ein Headset 14 (Kopfhörer und Mikrofon) angeschlossen werden. Die Verbindung mit Videokamera 13 und Headset 14 kann wieder drahtgebunden oder drahtlos erfolgen. Die Energieversorgung des Telemedizinmoduls 8 erfolgt vorzugsweise autark über eine wieder aufladbare Batterie, die vorzugsweise eine hinreichende Betriebszeit von wenigstens einigen Stunden ermöglicht.

Das Telemedizinmodul 8 weist vorzugsweise eine interne Datenbank und einen geeigneten Rechner auf, der dem Kabinenpersonal Hilfe im Umgang mit medizinischen Zwischen- und Notfällen auch dann leisten kann, wenn das Telemedizinmodul 8 autark und ohne Datenverbindung zu einer Bodenstation 6 arbeitet. Beispielsweise können mittels gespeicherter Anweisungen, Videosequenzen oder dergleichen Maßnahmen zum Anbringen von oder Umgang mit Sensoren, Maßnahmen zur Sicherstellung von Vitalfunktionen oder dergleichen vorgeschlagen werden. Ferner kann das Telemedizinmodul 8 anhand der über die Sensoren erfassten oder vom Kabinenpersonal über den Touchscreen 12 manuell eingegebenen medizinischen Daten in der Datenbank gespeicherte Vorschläge zum weiteren Vorgehen unterbreiten.

Vorzugsweise wird das Telemedizinmodul 8 jedoch nicht autark betrieben, sondern über das WLAN 2 und die Satellitenverbindung 3, 4, 5 eine Datenverbindung mit der Bodenstation 6 hergestellt. Diese wiederum sorgt vorzugsweise über das Internet 7 für eine Datenverbindung zu einer bei 15 schematisch angedeuteten Telemedizindienstleistungsstation, die mit wenigstens einem Arzt besetzt ist.

Vorzugsweise erfolgt die Datenverbindung zwischen dem Telemedizinmodul 8 und der Telemedizindienstleistungsstation 15 verschlüsselt, soweit zur Herstellung der Datenverbindung für Dritte zugängliche Datenverbindungen oder Netzwerke verwendet werden. Nach Herstellung der Verbindung können beispielsweise zunächst vom Telemedizinmodul 8 erfasste medizinische Daten an den Arzt in der Telemedizinstation 15 durchgegeben und dort ausgewertet werden. Mittels der Kamera 13 kann sich der Arzt in der Telemedizinstation 15 den Zustand des Patienten zeigen lassen oder die korrekte Ausführung diagnostischer oder therapeutischer Maßnahmen durch das Kabinenpersonal optisch überprüfen. Über das Headset 14 können Audioinformationen ausgetauscht werden.

Ist eine Videoübertragung nicht vorgesehen oder nicht mehr nötig, kann das Telemedizinmodul 8 in die zweite Betriebsart wechseln. In dieser Betriebsart wird über die gesamte Übertragungskette bis zur Telemedizinstation 15 entweder nur noch eine deutlich geringere Bandbreite vorgehalten, alternativ kann eine diskontinuierliche Datenübertragung im packet mode erfolgen, insbesondere dann, wenn lediglich vom Telemedizinmodul 8 erfasste Vitaldaten des Patienten laufend an die Telemedizinstation 15 übermittelt werden sollen. Da diese Daten im Telemedizinmodul 8 zwischengespeichert werden, wird diese in der Regel nicht zeitkritische Datenübertragung durch eine Datenübertragung im packet mode nicht beeinträchtigt.

Das Telemedizinmodul 8 weist bevorzugt einen Labeldrucker auf, der ein bei 16 angedeutetes Labelband ausdrucken kann, das den Patienten beispielsweise um das Handgelenk gelegt werden kann. Das ausgedruckte Label dient zur Identifizierung des Datensatzes, der über den medizinischen Zwischenfall erstellt und in einer Datenbank der Telemedizinstation 15 am Boden vorgehalten wird. Wird der Patient nach der Landung des Flugzeuges beispielsweise in ein Krankenhaus eingeliefert, kann dieses mit einem entsprechenden Lesegerät den Code des Labels 16 lesen und bekommt auf diese Weise einen autorisierten Zugang zu dem entsprechenden Datensatz in der Datenbank der Telemedizinstation 15. Die entsprechenden Daten über den Zwischenfall stehen dem Krankenhaus dann unmittelbar zur Verfügung, gleichzeitig wird ein unautorisierter zugriff von Dritten erschwert.

Die Datenverbindung zwischen dem Telemedizinmodul 8 und der Bodenstation 6 sowie der Telemedizinstation 15 kann während oder außerhalb von medizinischen Zwischenfällen auch dazu genutzt werden, einen Selbsttest des Telemedizinmoduls 8 durchzuführen, ein Softwareupdate durchzuführen oder dergleichen.

## Patentansprüche

1. Vorrichtung zum Erfassen und/oder Auswerten medizinischer Daten an Bord eines Verkehrsmittels (1), mit den Merkmalen:
a) wenigstens eine Schnittstelle für die Erfassung und/oder Eingabe medizinischer Daten;
b) eine Einrichtung zur Übermittlung medizinischer Daten und/oder Audio- und/oder Videodaten von der Vorrichtung an eine und/oder von einer außerhalb des Verkehrsmittels (1) befindliche/n Bodenstation (6);
**gekennzeichnet durch** folgende Merkmale:
c) die Vorrichtung (8) weist eine erste Betriebsart zur Übermittlung medizinischer Daten und/oder Audio- und/oder Videodaten von der Vorrichtung an eine und/oder von einer außerhalb des Verkehrsmittels befindliche/n Bodenstation (6) auf, in der sie zur Sicherung einer für eine Videoübertragung ausreichenden Bandbreite auf den verwendeten Datenübertragungsmitteln (2, 3, 4, 5) ausgebildet ist;
d) die Vorrichtung weist eine zweite Betriebsart zur Übermittlung medizinischer Daten und/oder Audio- und/oder Videodaten von der Vorrichtung an eine und/oder von einer außerhalb des Verkehrsmittels (1) befindliche/n Bodenstation (6) auf, in der sie zur Datenübertragung eine schmalere Bandbreite als in der ersten Betriebsart und/oder eine diskontinuierliche Datenübertragungsart (packet mode) verwendet.

2. Vorrichtung nach Anspruch 1, **dadurch gekennzeichnet, dass** die Schnittstellen für die Erfassung und/oder Eingabe medizinischer Daten Anschlüsse für Sensoren (9, 10, 11) für Vitalfunktionen des Körpers umfassen.

3. Vorrichtung nach Anspruch 1 oder 2, **dadurch gekennzeichnet, dass** die Schnittstellen für die Erfassung und/oder Eingabe medizinischer Daten (12, 13, 14) zur Erfassung von Audio- und/oder Videodaten und/oder zur Eingabe von Daten durch eine Hilfsperson ausgebildet sind.

4. Vorrichtung nach einem der Ansprüche 1 bis 3, **dadurch gekennzeichnet, dass** die Einrichtung zur Übermittlung medizinischer Daten und/oder Audio- und/oder Videodaten von der Vorrichtung an eine und/oder von einer außerhalb des Verkehrsmittels befindliche/n Bodenstation einen drahtlosen und/oder drahtgebunden Netzwerkanschluss zur Verbindung mit einem Netzwerk (2) des Verkehrsmittels (1) umfasst.

5. Vorrichtung nach einem der Ansprüche 1 bis 4, **dadurch gekennzeichnet, dass** in der ersten Betriebsart eine Bandbreite von wenigstens 64 kBit/s, vorzugsweise 64 bis 128 kBit/s, weiter vorzugsweise wenigstens 96 kBit/s vorgesehen ist.

6. Vorrichtung nach einem der Ansprüche 1 bis 5, **dadurch gekennzeichnet, dass** in der zweiten Betriebsart eine Bandbreite von wenigstens 16 kBit/s, vorzugsweise wenigstens 32 kBit/s, weiter vorzugsweise 32 bis 64 kBit/s vorgesehen ist.

7. Vorrichtung nach einem der Ansprüche 1 bis 6, **dadurch gekennzeichnet, dass** sie die erste Betriebsart automatisch einschaltet, wenn eine Videoübertragung vorgenommen wird.

8. Vorrichtung nach einem der Ansprüche 1 bis 7, **dadurch gekennzeichnet, dass** sie eine Identifizierungseinrichtung aufweist, die sie bei Verwendung der ersten Betriebsart bei dem Betreiber der verwendeten Datenübertragungsmittel (2, 3, 4, 5) als zur bevorrechtigten Anforderung einer entsprechenden Bandbreite identifiziert.

9. Vorrichtung nach einem der Ansprüche 1 bis 8, **dadurch gekennzeichnet, dass** sie zusätzlich eine dritte Betriebsart aufweist, in der keine Datenübertragung von einer/an eine Bodenstation (6) stattfindet.

10. Vorrichtung nach einem der Ansprüche 1 bis 9, **dadurch gekennzeichnet, dass** sie zusätzlich einen Labeldrucker (16) aufweist.

11. Anordnung zum Erfassen und/oder Auswerten medizinischer Daten an Bord eines Verkehrsmittels (1), **dadurch gekennzeichnet, dass** sie aufweist:
a) an Bord des Verkehrsmittels (1) eine Vorrichtung nach einem der Ansprüche 1 bis 10;
b) an Bord des Verkehrsmittels (1) ein Netzwerk (2) zur Übertragung der Daten von der Vorrichtung an eine Sende-/Empfangseinrichtung des Verkehrsmittels (1) und umgekehrt;
c) eine Bodenstation (6) mit einer Sende-/Empfangseinrichtung, die zur Datenkommunikation mit der Sende-/Empfangseinrichtung des Verkehrsmittels (1) ausgebildet ist;
d) in der Bodenstation Einrichtungen zur Datenkommunikation mit einer Einrichtung (15) zur Erbringung telemedizinischer Dienstleistungen.

12. Anordnung nach Anspruch 11, **dadurch gekennzeichnet, dass** das Netzwerk an Bord des Verkehrsmittels ein drahtloses Netzwerk, vorzugsweise ein W-LAN (2) ist.

13. Anordnung nach Anspruch 11 oder 12, **dadurch gekennzeichnet, dass** die Datenkommunikation zwischen der Sende-/Empfangseinrichtung des Verkehrsmittels (1) und der Bodenstation mittels einer Satellitenverbindung (3, 4, 5) erfolgt; und/oder dass die Datenkommunikation mit einer Einrichtung zur Erbringung telemedizinischer Dienstleistungen (15) über das Internet (7) erfolgt.

14. Anordnung nach Anspruch 12 oder 13, **dadurch gekennzeichnet, dass** die Datenkommunikation zwischen der Sende-/Empfangseinrichtung des Verkehrsmittels und der Bodenstation und/oder die Datenkommunikation mit einer Einrichtung zur Erbringung telemedizinischer Dienstleistungen verschlüsselt erfolgt.

15. Anordnung nach einem der Ansprüche 11 bis 14, **dadurch gekennzeichnet, dass** sie zusätzlich eine Datenbank aufweist, in der übermittelte medizinische Daten gespeichert sind, wobei diese Datenbank für über ein Autorisierungsmittel verfügende Dritte über ein Datennetzwerk, vorzugsweise über das Internet, zugänglich ist.
